# FASCICULE DE BREVET EUROPEEN

(11) **EP 1 647 303 B1**
(45) Date de publication et mention de la délivrance du brevet: **02.05.2007**
(21) Numéro de dépôt: 05292097.2
(22) Date de dépôt: 10.10.2005
(51) Int. Cl.: A61N 1/378

(54) **Dispositf médical implantable actif comprenant un circuit de télémétrie RF**
Implantierbare aktive medizinische vorrichtung mit telemetrischem RF-Schaltkreis
Implantable active medical system with telemetric RF-circuit

(30) Priorité: 12.10.2004 FR 0410742
(43) Date de publication de la demande: 19.04.2006
(73) Titulaire: ELA MEDICAL, 92541 Montrouge (FR)
(72) Inventeur: Dal Molin, Renzo, 92320 Châtillon (FR)
(74) Mandataire: Dupuis-Latour, Dominique

(56) Documents cités:
- EP-A- 1 062 983
- US-A- 5 807 397
- US-A1- 2002 068 957
- US-B1- 6 426 628

## Description

L'invention concerne les "dispositifs médicaux implantables actifs" tels que définis par la directive 90/385/CEE du 20 juin 1990 du Conseil des communautés européennes.

Cette définition inclut notamment les appareils chargés de surveiller l'activité cardiaque et de générer des impulsions de stimulation, de resynchronisation, de défibrillation et/ou de cardioversion en cas de trouble du rythme détecté par l'appareil. Elle inclut aussi les appareils neurologiques, les pompes de diffusion de substances médicales, les implants cochléaires, les capteurs biologiques implantés, etc., ainsi que les dispositifs de mesure de pH ou encore d'impédance intracorporelle (telle que mesure d'impédance transpulmonaire ou d'impédance intracardiaque).

Avec ces dispositifs, il est possible d'opérer un échange de données avec un "programmateur", qui est un dispositif externe permettant de vérifier le paramétrage du dispositif, de lire des informations enregistrées par celui-ci ou d'y inscrire des informations, ou encore de mettre à jour le logiciel interne de pilotage du dispositif.

Cet échange de données est effectué par télémétrie, c'est-à-dire par une technique de transmission d'informations à distance, sans contact galvanique. Jusqu'à présent, la télémétrie était essentiellement réalisée par couplage magnétique entre des bobines du dispositif implanté et du programmateur, technique connue sous le nom de "procédé par induction". Cette technique présente cependant l'inconvénient, en raison de la très faible portée d'un couplage inductif, de nécessiter l'utilisation d'une "tête de télémétrie" contenant une bobine à placer au voisinage du site d'implantation du dispositif.

Il a été récemment proposé de mettre en oeuvre une autre technique de couplage non galvanique, utilisant les deux composantes d'une onde électromagnétique produite par des circuits émetteurs/récepteurs opérant dans le domaine des radiofréquences (RF), typiquement des fréquences de l'ordre de quelques centaines de mégahertz. Cette technique, dite de "télémétrie RF" permet de programmer ou interroger des implants à des distances supérieures à 3 m, et autorisent donc l'échange d'informations sans manipulation d'une tête de télémétrie, et même sans intervention d'un opérateur externe.

Les US-A-2003/0114897 et US-A-2003/0149459 décrivent de tels implants et programmateurs pourvus de circuits de télémétrie RF.

Les circuits RF de l'implant nécessitent cependant un courant d'alimentation très supérieur à ce qui est nécessaire aux autres circuits de l'implant (circuits de détection et de stimulation, principalement), la consommation d'un circuit RF pouvant dépasser 3 mA pendant les phases d'émission.

Dans le cas des défibrillateurs, compte tenu des appels de courant importants requis par les circuits d'application de la thérapie de choc, les piles utilisées sont à faible résistance interne et peuvent débiter sans difficulté des courants de l'ordre de quelques milliampères.

En revanche, les stimulateurs et dispositifs analogues tels que dispositifs multisite ou resynchroniseurs sont généralement alimentés par des piles de petite dimension de type lithium-iode ou équivalent, compte tenu du faible courant de fonctionnement requis par les circuit de détection et de stimulation. Ces piles présentent une résistance interne de l'ordre de 100 Ω en début de vie, qui augmente très vite à des valeurs bien supérieures, de l'ordre de 1 kΩ, 2 kΩ et même plus au fur et à mesure de la décharge de la pile. Cette résistance interne n'est pas gênante pour les circuits à faible consommation, mais elle empêche d'alimenter convenablement des circuits RF avec le niveau de courant requis.

Une première solution consiste à utiliser un autre type de pile, par exemple une pile bouton soudable de faible impédance lithium-manganèse (LiMnO₂) de taille réduite. Il existe de telles piles dont les caractéristiques sont : diamètre 12,2 mm, hauteur 1,4 mm, capacité 27 mAh, tension nominale 3 V, autodécharge maximale 1 % par an, et qui peuvent fournir des courants de plusieurs milliampères. Il est possible d'utiliser des piles réalisées à partir d'autres couples, ayant une densité volumique semblable ou meilleure, une faible autodécharge, pouvant fournir 3mA ou plus. Le courant des circuits RF est fourni par la pile bouton exclusivement ; quand la pile bouton ne peut plus fournir le courant, la sauvegarde est assurée par la pile lithium du pacemaker qui fournit un courant faible de 10µA ce qui permet un débit de 1,7 koctets/s., l'émetteur-récepteur travaillant en mode pulsé. Le courant de pointe est fournit par un condensateur faisant partie d'une alimentation régulant la tension de la pile.

Une autre solution est proposée par le US-A-5 807 397, qui divulgue un implant de neurostimulation dépourvue de pile d'alimentation, mais où l'énergie est délivrée par un condensateur de forte valeur rechargé à intervalles réguliers par un module extérieur, couplé à l'implant par voie inductive. Des moyens de télémétrie permettent notamment d'interroger l'implant pour connaître le degré de charge du condensateur.

Le US-A-2002/006 8957 décrit également un implant de stimulation des tissus corporels alimenté par une batterie rechargeable de l'extérieur par voie inductive (mais dépourvu de moyens de télémétrie).

Les caractéristiques techniques mentionnées dans la première partie de la revendication 1 sont toutes divulguées dans l'état de la technique cité.

L'invention propose une autre solution au problème précité, qui ne nécessite pas le recours à une pile supplémentaire, grâce à un circuit permet-tant de fournir à un circuit de télémétrie RF incorporé à un implant le courant élevé nécessaire au fonctionnement de celui-ci, ceci à partir d'une pile conventionnelle et pendant toute la durée de vie de cette pile.

À cet effet, le dispositif de l'invention, qui comprend un circuit principal, un circuit auxiliaire de télémétrie RF et une pile d'alimentation des circuits principal et auxiliaire, comporte entre la pile d'alimentation et le circuit auxiliaire un circuit régulateur comprenant un organe accumulateur d'énergie électrique, couplé au circuit auxiliaire pour délivrer un courant apte à alimenter ce circuit auxiliaire, et un circuit de charge de l'organe accumulateur, couplé à la pile d'alimentation pour amener et maintenir cet organe accumulateur à un niveau de charge prédéterminé.

L'organe accumulateur peut être une batterie rechargeable, ou un condensateur.

Lorsque la tension correspondant au niveau de charge prédéterminé est supérieure à la tension délivrée par la pile d'alimentation, le circuit de charge comprend un étage multiplicateur de tension.

Avantageusement, le circuit de charge de l'organe accumulateur est un circuit à fonctionnement intermittent et cyclique, avec alternance de cycles d'alimentation du circuit régulateur et de cycles d'alimentation du circuit principal. Le rapport cyclique peut être un rapport variable fonction de la résistance interne de la pile d'alimentation, la durée relative des cycles d'alimentation du circuit régulateur diminuant lorsqu'augmente ladite résistance interne, et/ou fonction du niveau de charge de l'organe accumulateur, la durée relative des cycles d'alimentation du circuit régulateur diminuant lorsqu'augmente ledit niveau de charge.

Le circuit de charge peut interrompre la charge de l'organe accumulateur lorsque le niveau de tension aux bornes de l'organe accumulateur atteint une limite haute prédéterminée, et/ou lorsque le courant de charge de l'organe accumulateur atteint une limite basse prédéterminée, et/ou après écoulement d'une durée de charge prédéterminée.

On va maintenant décrire un exemple de mise en oeuvre du dispositif de l'invention, en référence aux dessins annexés où les mêmes références numériques désignent d'une figure à l'autre des éléments identiques.
La figure 1 est un schéma synoptique simplifié des différents éléments constituant le circuit d'alimentation selon l'invention.
La figure 2 montre le détail du multiplicateur de tension utilisé par le circuit de la figure 1.
Les figures 3 et 4 montrent les deux configurations, charge et décharge, du multiplicateur de tension de la figure 2 en fonction des états de commutation des divers interrupteurs.

On va maintenant décrire un exemple de réalisation du dispositif de l'invention. Celle-ci peut notamment être appliquée aux dispositifs implantables commercialisés par ELA Médical, Montrouge, France tels que les appareils *Symphony* et *Rhapsody.* Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. L'adaptation de ces appareils à la mise en oeuvre des fonctions de l'invention est à la portée de l'homme du métier, et elle ne sera pas décrite en détail (en ce qui concerne ses aspects logiciels, l'invention peut être mise en oeuvre par une programmation appropriée du logiciel de commande du stimulateur).

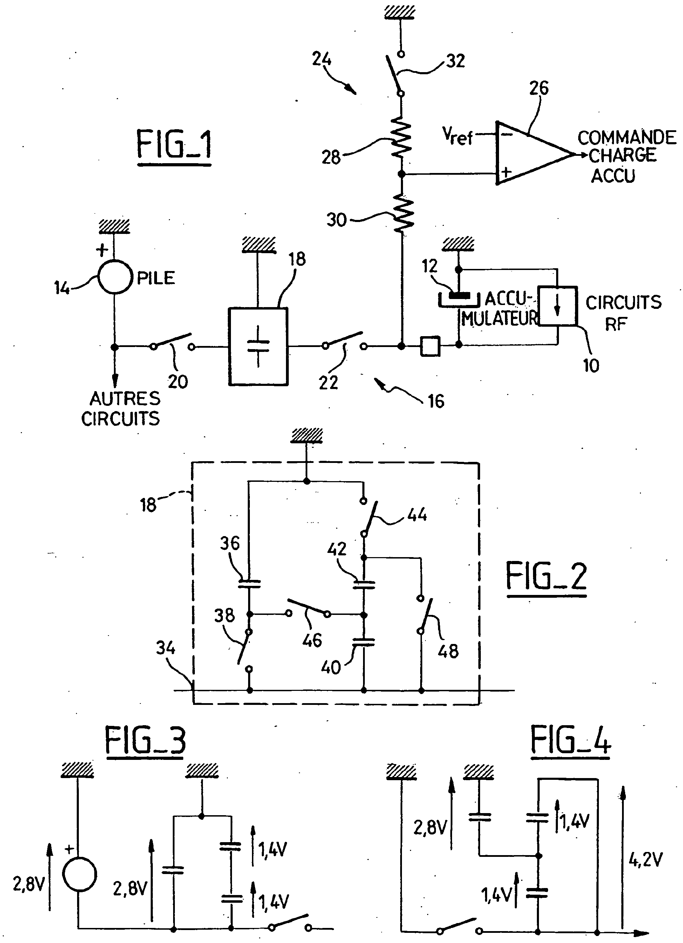

Sur la figure 1, la référence 10 désigne de façon générale les circuits de télémétrie RF, auxquels il est nécessaire de fournir un courant d'alimentation relativement élevé (plusieurs milliampères), notamment lors des phases d'émission du signal modulé.

Pour délivrer un tel courant d'alimentation, l'invention propose d'alimenter ces circuits RF à partir d'un accumulateur tampon 12, lui-même chargé par la pile d'alimentation 14 du dispositif implanté au moyen d'un circuit régulateur 16. La pile alimente également, d'une manière non modifiée, les autres circuits du dispositif (détection, stimulation, ...).

L'accumulateur 12 peut être un accumulateur de type lithium-ion, dont il existe des modèles de taille réduite présentant des caractéristiques compatibles avec les besoins en alimentation de circuit RF d'un dispositif implanté, typiquement : capacité 10 mA.h, résistance interne 25 Ω en continu et 8 Ω en alternatif, autodécharge maximale de 15% par an, rechargeable 250 fois avec une perte maximale de capacité de 14%. De tels accumulateurs sont notamment fabriqués par la société Quallion LLC, Sylmar, Californie, USA. En variante, l'accumulateur lithium-ion peut être remplacé par un condensateur de très forte capacité nominale, typiquement de l'ordre de 1 farad.

Les accumulateurs lithium-ion présentent une tension nominale de 4 V à pleine charge, qui peut ensuite descendre à une valeur de l'ordre de 3 V. Comme les piles lithium-iode utilisées dans les stimulateurs cardiaques ont une tension nominale de l'ordre de 2,8 V, cette tension est insuffisante pour charger l'accumulateur et il est nécessaire de prévoir un étage intermédiaire 18 multiplicateur de tension permettant de délivrer à l'accumulateur une tension de charge de 2,8 x 1,5 = 4,2 V.

Ce multiplicateur de tension 18 est relié d'une part à la pile par un interrupteur 20 et d'autre part à l'accumulateur par un interrupteur 22. Son fonctionnement, et donc la charge de l'accumulateur, sont contrôlés par un circuit de commande 24 comprenant un comparateur 26 dont une entrée est reliée à un tension de référence V_{ref} et l'autre entrée est reliée au point milieu d'un diviseur de tension résistif 28, 30 donnant une image de la tension aux bornes de l'accumulateur 12 et mis en service par fermeture d'un interrupteur 32.

La structure interne du multiplicateur de tension 18 est illustrée figure 2 : celui-ci comprend une entrée 34 reliée via l'interrupteur 20 à la pile 14 permettant de charger un premier condensateur 36 par fermeture d'un interrupteur 38. Cette même entrée permet également de charger deux condensateurs 40, 42 montés en série, par fermeture d'un interrupteur 44. Par ailleurs, un interrupteur 46 permet de relier le point milieu des condensateurs 40, 42 au point commun du condensateur 36 et de l'interrupteur 38. Enfin, un interrupteur 48 permet de court-circuiter l'ensemble formé par les condensateurs 40 et 42.

Dans la phase initiale, correspondant à la configuration de la figure 3, les commutateurs 20, 38 et 44 sont fermés, tandis que les commutateurs 22, 46 et 48 sont ouverts : le condensateur 36 est ainsi chargé à la tension de la pile (2,8 V) et les condensateurs 40 et 42 sont chacun chargés à la moitié de cette tension (1,4 V). Dans la phase suivante, les commutateurs 20, 38 et 44 sont ouverts, tandis que les commutateurs 22, 46 et 48 sont fermés : les condensateurs 40 et 42 se retrouvent alors mis en parallèle, et la tension à leurs bornes (1,4 V) vient s'ajouter à celle (2,8 V) aux bornes du condensateur 36, donnant ainsi en sortie une tension de 2,8 + 1,4 = 4,2 V.

Cette tension de 4,2 V produite par le multiplicateur de tension 18 est utilisée pour charger l'accumulateur 12, avec un courant de charge pouvant par exemple varier de 2 à 0,1 mA, en fonction de l'évolution de la résistance interne de la pile 14.

Avantageusement, cette charge de l'accumulateur n'est pas opérée de façon permanente, mais de façon intermittente et cyclique, par exemple avec une charge pendant 25% d'un cycle de 1 seconde, les 75% restants étant consacrés à l'alimentation des autres circuits (détection, stimulation, ...) du dispositif.

Avantageusement, le rapport cyclique (25% dans l'exemple ci-dessus) est un rapport variable, fonction de la résistance interne de la pile d'alimentation 14 (la durée des phases de charge devenant plus courte lorsque la résistance interne augmente) et/ou du niveau de charge de l'accumulateur 12 (la durée des cycles de charge diminuant au fur et à mesure que l'accumulateur se rapproche de son niveau de charge maximale).

La charge de l'accumulateur se poursuit ainsi jusqu'à atteindre un niveau prédéterminé, par exemple lorsque le circuit de contrôle de charge 26 détecte que la tension aux bornes de l'accumulateur atteint 4 V à vide. Le comparateur 26 bascule alors pour suspendre la charge jusqu'à ce que la tension aux bornes de l'accumulateur vienne à descendre au-dessous d'un seuil donné du fait de la consommation d'énergie des circuits RF.

La charge peut être également interrompue en fonction d'autres critères, par exemple lorsque le courant de charge atteint une limite basse du fait de l'accumulateur, ou encore par sécurité au bout d'une durée maximale donnée, par exemple au bout de 100 heures pour 10 mA de courant de charge.

Les circuits RF 10, alimentés par l'énergie stockée dans l'accumulateur 10, pourront être alimentés de manière satisfaisante avec un courant débité relativement important, par exemple de 3 à 20 mA.

Pour tenir compte de la différence entre la tension aux bornes de l'accumulateur (de l'ordre de 3 à 4 V selon le niveau de charge) et le niveau de tension nominal requis pour l'alimentation des composants RF (typiquement entre 1,8 et 3 V), il est prévu un régulateur approprié, par exemple un régulateur linéaire ou selfique, pour générer la tension d'alimentation voulue avec une capacité en courant appropriée.

## Revendications

1. Un dispositif médical implantable actif, notamment un dispositif de stimulation, resynchronisation, défibrillation et/ou cardioversion, comprenant un circuit principal, un circuit auxiliaire (10) de télémétrie RF et une pile (14) d'alimentation des circuits principal et auxiliaire,
**caractérisé en ce qu'**il est prévu entre la pile d'alimentation et le circuit auxiliaire un circuit régulateur (16) comprenant :
- un organe accumulateur d'énergie électrique (12), couplé au circuit auxiliaire (10) pour délivrer un courant apte à alimenter ce circuit auxiliaire, et
- un circuit (18, 20, 22, 24) de charge de l'organe accumulateur, couplé à la pile d'alimentation (14) pour amener et maintenir cet organe accumulateur (12) à un niveau de charge prédéterminé.

2. Le dispositif de la revendication 1, où ledit organe accumulateur (12) est une batterie rechargeable.

3. Le dispositif de la revendication 1, où ledit organe accumulateur (12) est un condensateur.

4. Le dispositif de la revendication 1, où la tension correspondant audit niveau de charge prédéterminé est supérieure à la tension délivrée par la pile d'alimentation, et où le circuit de charge de l'organe accumulateur comprend un étage (18) multiplicateur de tension.

5. Le dispositif de la revendication 1, où le circuit de charge de l'organe accumulateur est un circuit à fonctionnement intermittent et cyclique, avec alternance de cycles d'alimentation du circuit régulateur et de cycles d'alimentation du circuit principal.

6. Le dispositif de la revendication 5, où le rapport cyclique du fonctionnement intermittent du circuit de charge est un rapport variable fonction de la résistance interne de la pile d'alimentation, la durée relative des cycles d'alimentation du circuit régulateur diminuant lorsqu'augmente ladite résistance interne.

7. Le dispositif de la revendication 5, où le rapport cyclique du fonctionnement intermittent du circuit de charge est un rapport variable fonction du niveau de charge de l'organe accumulateur, la durée relative des cycles d'alimentation du circuit régulateur diminuant lorsqu'augmente ledit niveau de charge.

8. Le dispositif de la revendication 1, où le circuit de charge comprend des moyens pour interrompre la charge de l'organe accumulateur lorsque le niveau de tension aux bornes de l'organe accumulateur atteint une limite haute prédéterminée.

9. Le dispositif de la revendication 1, où le circuit de charge comprend des moyens pour interrompre la charge de l'organe accumulateur lorsque le courant de charge de l'organe accumulateur atteint une limite basse prédéterminée.

10. Le dispositif de la revendication 1, où le circuit de charge comprend des moyens pour interrompre la charge de l'organe accumulateur après écoulement d'une durée de charge prédéterminée.

## Claims

1. An active implantable medical device, such as a stimulation, resynchronisation, defibrillation, and/or cardioversion device, comprising a main circuit, an auxiliary RF telemetry circuit (10), and a battery (14) for supplying the main and auxiliary circuits,
**characterised in that** there is provided a regulating circuit (16) between the supply battery and the auxiliary circuit, comprising:
- an electric energy accumulator (12), coupled with the auxiliary circuit (10) to deliver a current able to power this auxiliary circuit; and
- an accumulator charging circuit (18, 20, 22, 24), coupled with the supply battery (14) to bring this accumulator (12) to and maintain it at a predetermined charge level.

2. The device of claim 1, wherein said accumulator (12) is a rechargeable battery.

3. The device of claim 1, wherein said accumulator (12) is a capacitor.

4. The device of claim 1, wherein the voltage corresponding to said predetermined charge level is higher than the voltage delivered by the supply battery, and wherein the accumulator charging circuit includes a voltage multiplying stage (18).

5. The device of claim 1, wherein the accumulator charging circuit is a circuit with intermittent and cyclic operation, with an alternation of regulating circuit supply cycles and main circuit supply cycles.

6. The device of claim 5, wherein the cyclic ratio of the intermittent operation of the charging circuit is a variable ratio, which is a function of the internal resistance of the supply battery, the relative duration of the regulating circuit supply cycles decreasing when the internal resistance increases.

7. The device of claim 5, wherein the cyclic ratio of the intermittent operation of the charging circuit is a variable ratio, which is a function of the charge level of the accumulator, the relative duration of the regulating circuit supply cycles decreasing when said charge level increases.

8. The device of claim 1, wherein the charging circuit comprises means for interrupting the accumulator charging when the voltage level at the accumulator terminals reaches a predetermined upper limit.

9. The device of claim 1, wherein the charging circuit comprises means for interrupting the accumulator charging when the accumulator charging current reaches a predetermined lower limit.

10. The device of claim 1, wherein the charging circuit comprises means for interrupting the accumulator charging after expiration of a predetermined charging duration.

## Patentansprüche

1. Aktive implantierbare medizinische Vorrichtung, insbesondere eine Vorrichtung zur Stimulation, Resynchronisation, Defibrillation, und/oder Kardioversion, mit einer Hauptschaltung, einer Hilfsschaltung (10) zur Radiofrequenztelemetrie und einer Batterie (14) zur Versorgung der Haupt- und Hilfsschaltungen,
**dadurch gekennzeichnet, dass** zwischen der Versorgungsbatterie und der Hilfsschaltung eine Steuerschaltung (16) vorgesehen ist, mit:
- einem Element zur Akkumulation elektrischer Energie (12), das mit der Hilfsschaltung (10) gekoppelt ist, um einen Stromfluss zu liefern, der geeignet ist, diese Hilfsschaltung zu versorgen, und
- einer Schaltung (18, 20, 22, 24) zur Aufladung des Akkumulationselements, die mit der Versorgungsbatterie (14) gekoppelt ist, um dieses Akkumulationselement (12) auf einen vorbestimmten Ladungspegel zu bringen und dort zu halten.

2. Vorrichtung nach Anspruch 1, bei welcher das Akkumulationselement (12) eine wieder aufladbare Batterie ist.

3. Vorrichtung nach Anspruch 1, bei welcher das Akkumulationselement (12) ein Kondensator ist.

4. Vorrichtung nach Anspruch 1, bei welcher die dem vorbestimmten Ladungspegel entsprechende Spannung größer als die durch die Versorgungsbatterie gelieferte Spannung ist, und bei welcher die Schaltung zur Aufladung des Akkumulationselements eine Spannungsvervielfachungsstufe (18) umfasst.

5. Vorrichtung nach Anspruch 1, bei welcher die Schaltung zur Aufladung des Akkumulationselements eine Schaltung mit zyklischem und zeitweiligem Betrieb ist, mit einem Wechsel von Zyklen zur Versorgung der Steuerschaltung und Zyklen zur Versorgung der Hauptschaltung.

6. Vorrichtung nach Anspruch 5, bei welcher das zyklische Verhältnis des zeitweiligen Betriebs der Aufladungsschaltung ein veränderliches Verhältnis ist, das vom Innenwiderstand der Versorgungsbatterie abhängt, wobei die relative Dauer der Zyklen zur Versorgung der Steuerschaltung sich verringert, wenn der Innenwiderstand sich erhöht.

7. Vorrichtung nach Anspruch 5, bei welcher das zyklische Verhältnis des zeitweiligen Betriebs der Aufladungsschaltung ein veränderliches Verhältnis ist, das vom Aufladungspegel des Akkumulationselements abhängt, wobei die relative Dauer der Zyklen zur Versorgung der Steuerschaltung sich verringert, wenn der Aufladungspegel sich erhöht.

8. Vorrichtung nach Anspruch 1, bei welcher die Aufladungsschaltung Mittel umfasst, um die Aufladung des Akkumulationselements zu unterbrechen, wenn das Spannungsniveau an den Anschlüssen des Akkumulationselements eine vorbestimmte obere Grenze erreicht.

9. Vorrichtung nach Anspruch 1, bei welcher die Aufladungsschaltung Mittel umfasst, um die Aufladung des Akkumulationselements zu unterbrechen, wenn der Strom zur Aufladung des Akkumulationselements eine vorbestimmte untere Grenze erreicht.

10. Vorrichtung nach Anspruch 1, bei welcher die Aufladungsschaltung Mittel umfasst, um die Aufladung des Akkumulationselements nach Ablauf einer vorbestimmten Aufladedauer zu unterbrechen.
